# EUROPÄISCHE PATENTSCHRIFT

(11) **EP 1 473 057 B1**
(45) Veröffentlichungstag und Bekanntmachung des Hinweises auf die Patenterteilung: **28.02.2007**
(21) Anmeldenummer: 04007898.2
(22) Anmeldetag: 01.04.2004
(51) Int. Cl.: A61N 5/06, H05B 3/00, H05B 3/44

(54) **Infrarotstrahler**
Infrared radiator
Radiateur infra-rouge

(30) Priorität: 29.04.2003 DE 10319468
(43) Veröffentlichungstag der Anmeldung: 03.11.2004
(73) Patentinhaber: Heraeus Noblelight GmbH, 63450 Hanau (DE)
(72) Erfinder: Grob, Siegfried, 63477 Maintal (DE); Linow, Sven, Dr., 63450 Hanau (DE); Scherzer, Joachim, 63486 Bruchköbel (DE)
(74) Vertreter: Kühn, Hans-Christian

(56) Entgegenhaltungen:
- EP-A- 0 446 458
- DE-A- 1 917 257
- US-A- 3 160 777
- US-A- 5 686 794
- US-A- 6 057 532

## Beschreibung

Die Erfindung betrifft einen Infrarotstrahler mit mindestens einem elektrischen Heizleiter, welcher in einem zweiendigen, langgestreckten Hüllrohr aus Kieselglas angeordnet ist, wobei das Hüllrohr an seiner dem mindestens einen Heizleiter zugewandten Wandung mindestens eine Erhebung aus Kieselglas aufweist, die den Innendurchmesser des Hüllrohres lokal vermindert.

Derartige Infrarotstrahler sind aus der US 6,057,532 bekannt. Es ist ein Infrarotstrahler mit einem Heizleiter aus Carbonfasern offenbart, der in einem Hüllrohr aus Kieselglas angeordnet ist. Zur Fixierung und Zentrierung von bandförmigen, langgestreckten Heizleitern im Hüllrohr sind Fixierungs-Mulden im Hüllrohr offenbart, die - im Querschnitt des Hüllrohres betrachtet-punktförmig und sich direkt gegenüberliegend an der Hüllrohrinnenwandung angeordnet sind. Der Außendurchmesser des Hüllrohrs ist dabei im Bereich der Fixierungs-Mulden lokal verändert. Die Fixierungs-Mulden weisen ein U-förmiges Profil auf, durch welches die Kanten eines bandförmigen Heizleiters zur Verhinderung eines Verdrehens des Bandes geführt werden.

Aus der EP 0 446 458 B1 ist eine Halogenglühlampe für die Allgemeinbeleuchtung offenbart, die einen ähnlichen Aufbau zeigt. Es ist ein langgestreckter, beidseitig verschlossener Kolben aus Kieselglas mit einem darin angeordneten Leuchtkörper offenbart, der durchgehend gewendelt sein kann. Zur Abstützung des Leuchtkörpers werden quer zur Lampenachse Stege in den Kolben eingebracht, wobei der Außendurchmesser des Kolbens lokal verändert wird.

Die Lampen gemäß der US 6,057,532 oder EP 0 446 458 B1 werden demnach durch ein zusätzliches, nachträgliches Verformen der Hüllrohr- oder Kolbenwandung im Bereich des Außendurchmessers ausgebildet. Derartige Vorgänge sind energieintensiv und bergen die Gefahr in sich, dass das Hüllrohr beziehungsweise der Kolben beim Verformen beschädigt wird. Unweigerlich auftretende Dickenänderungen in der Wandung des Hüllrohres können zu Undichtigkeiten oder gar zum Bruch der Lampe führen.

Es ist nun Aufgabe der Erfindung, einen Infrarotstrahler mit einer Emissionstemperatur des Heizleiters im Bereich von ≥ 1000°C, vorzugsweise im Bereich von 1000°C bis 1250°C, bereitzustellen, der stabil und dennoch kostengünstig hergestellt werden kann.

Die Aufgabe wird dadurch gelöst, dass mindestens ein Heizleiter als eine Heizwendel ausgebildet ist, das Hüllrohr im Bereich der Heizwendel einen konstanten Außendurchmesser aufweist, die Erhebung auf ihrer der Heizwendel zugewandten Oberfläche konvex geformt ist und die Heizwendel die Erhebung lediglich an der höchsten Stelle der jeweiligen Erhebung berührt.

Eine derartige Anordnung führt zu einem lediglich punkt- oder linienförmigen Kontakt zwischen der Heizwendel und dem Hüllrohr, wobei keine Veränderungen des Außendurchmessers des Hüllrohres notwendig sind. Die Heizwendel kann dabei ohne zusätzliche Einbauteile wie zum Beispiel Abstandshalter direkt in das Hüllrohr gelegt werden.
Eine Anfälligkeit des Infrarotstrahlers für Bruch ist aufgrund einer im wesentlichen gleichbleibenden Wandstärke minimiert. Zudem wird die Wärmeleitung von der Heizwendel auf das Hüllrohr minimiert, so dass eine Entglasung des Hüllrohres erst bei Temperaturen auftritt, die um ca. 200°C über der kritischen Temperatur liegen, die bei einem flächigen Kontakt von Heizwendel und Hüllrohr zur Entglasung des Hüllrohres führt. Überraschender Weise hat sich gezeigt, dass es bei dem erfindungsgemäßen Infrarotstrahler aufgrund des lediglich punkt- oder linienförmigen Kontakts zwischen Hüllrohr und Heizwendel auch ohne eine zusätzliche Kühlung des Hüllrohres nicht zu einer Entglasung des Hüllrohres kommt, wenn die Heizwendel eine Temperatur oberhalb der kritischen Temperatur erreicht. Ursache dafür ist, dass die Wärmeableitung durch das Hüllrohr und die Wärmeabstrahlung des Hüllrohres selbst eine ausreichende Kühlung der Erhebungen ermöglichen, so dass eine Entglasung entweder gar nicht auftritt oder - falls eine Entglasung an den Kontaktstellen zwischen Heizwendel und Erhebungen auftritt - sich diese nicht über die gesamte Erhebung ausbreiten kann.
Der Infrarotstrahler ist so schneller, kostengünstiger und mit geringeren Ausschussraten herzustellen.

Es hat sich bewährt, wenn die Erhebung(en) mindestens eine Linie von einem Ende des Hüllrohres zum anderen Ende des Hüllrohres beschreibt / beschreiben.
Dabei kann die mindestens eine Linie aus einer einzigen, langgestreckten Erhebung gebildet sein. Die linienförmige Erhebung kann dabei einen gleichbleibenden oder aber einen sich ändernden Querschnitt aufweisen.
Die mindestens eine Linie kann aber auch aus einer Anreihung von einzelnen Erhebungen gebildet sein. Die linienförmige Erhebung weist dabei vorzugsweise einen gleichbleibenden Querschnitt auf.

Es hat sich dabei bewährt, wenn im Querschnitt des Hüllrohres gesehen mindestens drei Erhebungen gleichmäßig über den Innendurchmesser des Hüllrohres verteilt angeordnet sind, so dass die Heizwendel nicht in Kontakt zu anderen Teilen des Hüllrohres als den Erhebungen kommen kann. Die maximale Anzahl an Erhebungen ist aus technischen sowie wirtschaftlichen Gründen begrenzt. Insbesondere hat es sich aber bewährt, wenn im Querschnitt des Hüllrohres gesehen 5 bis 6 Erhebungen gleichmäßig über den Innendurchmesser des Hüllrohres verteilt angeordnet sind.

Weiterhin hat es sich bewährt, wenn das Hüllrohr eine Längsachse aufweist und die mindestens eine Linie parallel zur Längsachse oder wendelförmig um die Längsachse des Hüllrohrs verläuft.

Das Hüllrohr kann in einer weiteren vorteilhaften Ausgestaltung auch eine Längsachse aufweisen, wobei die Erhebung(en) mindestens eine kreisförmige Linie um die Längsachse des Hüllrohrs beschreibt / beschreiben. Vorteilhafter Weise ist die mindestens eine kreisförmige Linie dabei aus einer einzigen, langgestreckten Erhebung gebildet. Die linienförmige Erhebung kann dabei einen gleichbleibenden oder einen sich ändernden Querschnitt aufweisen.
Es hat sich aber auch bewährt, wenn die mindestens eine Linie aus einer Anreihung von einzelnen Erhebungen gebildet ist. Vorteilhafter Weise weisen die linienförmigen Erhebungen dann einen gleichbleibenden Querschnitt auf.

Es ist bevorzugt, wenn der Innendurchmesser des Hüllrohres im Bereich der Erhebungen um etwa 10% bis 20% reduziert ist. Bei einem Innendurchmesser eines im wesentlichen zylindrischen Hüllrohres mit kreisringförmigem Querschnitt von 18mm wären beispielsweise Erhebungen im Bereich von etwa 1 mm vorteilhaft, so dass der Innendurchmesser im Bereich der Erhebungen auf circa 16mm reduziert wird.

Der Einsatz einer Heizwendel aus Wolframdraht, einem Kohlenstoff-Fasermaterial, Graphit, Graphitpapier, einer Eisen-Chrom-Aluminium-Legierung, einer Nickel-Chrom-Legierung oder einer Nickel-Chrom-Eisen-Legierung hat sich bewährt.

Wenn die Heizwendel aus Wolframdraht, einem Kohlenstoff-Fasermaterial, Graphit oder Graphitpapier gebildet ist, hat es sich bewährt, wenn das Hüllrohr an seinen Enden derart verschlossen ist, dass es den mindestens einen elektrischen Heizleiter gasdicht umschließt.

Wenn die Heizwendel aus einer Eisen-Chrom-Aluminium-Legierung, einer Nickel-Chrom-Legierung oder einer Nickel-Chrom-Eisen-Legierung bebildet ist, hat es sich bewährt, wenn das Hüllrohr an mindestens einem seiner Enden offen ist, so dass eine oxidierende Atmosphäre den mindestens einen elektrischen Heizleiter umgibt.

Weiterhin hat es sich als günstig erwiesen, wenn das Hüllrohr als ein Zwillingsrohr mit zwei Rohrkanälen ausgebildet ist, wobei das Hüllrohr in mindestens einem der beiden Rohrkanäle eine Heizwendel aufweist.

Die Figuren 1 bis 6a sollen den erfindungsgemäßen Infrarotstrahler beispielhaft erläutern. So zeigt:
- Figur 1: einen Längsschnitt durch einen erfindungsgemäßen Infrarotstrahler,
- Figur 1a: den Querschnitt A - A' durch den Infrarotstrahler aus Fig. 1,
- Figur 2: einen Längsschnitt durch ein für einen erfindungsgemäßen Infrarotstrahler geeignetes Hüllrohr,
- Figur 2a: den Querschnitt B - B' durch das Hüllrohr aus Fig. 2,
- Figur 3: einen Längsschnitt durch ein für einen erfindungsgemäßen Infrarotstrahler geeignetes Hüllrohr,
- Figur 3a: den Querschnitt C - C' durch das Hüllrohr aus Fig. 3,
- Figur 4: einen Längsschnitt durch ein für einen erfindungsgemäßen Infrarotstrahler geeignetes Hüllrohr,
- Figur 4a: den Querschnitt D - D' durch das Hüllrohr aus Fig. 4,
- Figur 5: einen Längsschnitt durch ein für einen erfindungsgemäßen Infrarotstrahler geeignetes Hüllrohr,
- Figur 5a: den Querschnitt E - E' durch das Hüllrohr aus Fig. 5,
- Figur 6: einen Längsschnitt durch einen erfindungsgemäßen Infrarotstrahler mit einem Zwillingsrohr als Hüllrohr,
- Figur 6a: den Querschnitt F - F' durch den Infrarotstrahler aus Fig. 6.

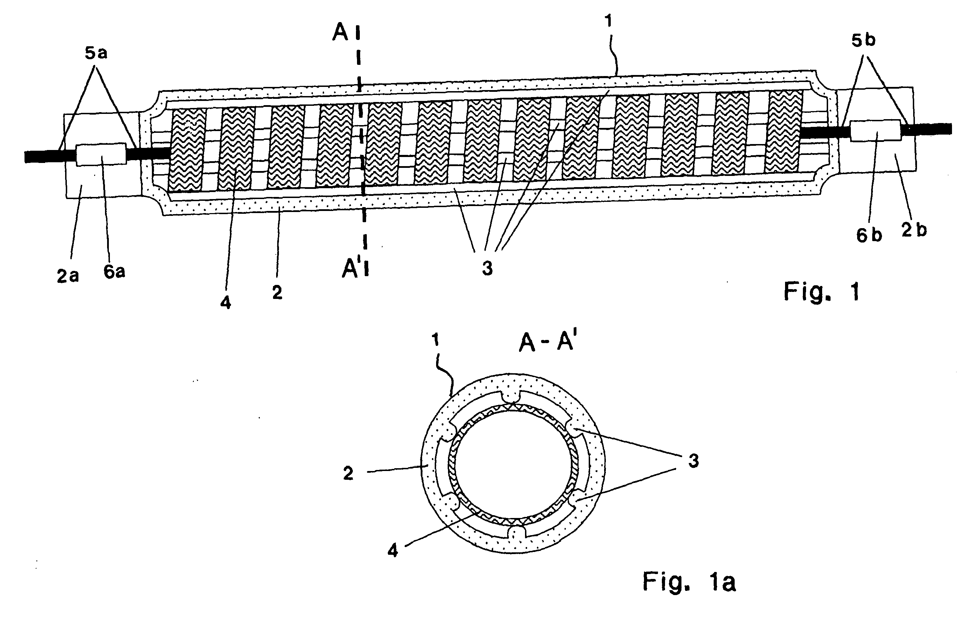

Figur 1 zeigt den Längsschnitt eines Infrarotstrahlers 1 mit einem Hüllrohr 2 aus Kieselglas, das einen Innendurchmesser von 18mm aufweist, der im Bereich der Erhebungen 3 auf 16mm reduziert ist. Das Hüllrohr 2 weist an seinem Innendurchmesser die Erhebungen 3 aus Kieselglas auf, die sich in gerader Linie von einem Ende des Hüllrohres 2 zum anderen Ende des Hüllrohres 2 erstrecken. Eine Heizwendel 4 aus Carbonfaser-Material ist im Hüllrohr 2 derart angeordnet, dass der Außendurchmesser der Heizwendel 4 die Erhebungen 3 lediglich an deren höchsten Stellen berühren kann. Dabei liegen allerdings bei einem Infrarotstrahler 1 in der Praxis nur vereinzelte Wendelbereiche der Heizwendel 4 auf Erhebungen 3 auf. Das Hüllrohr 2 ist an seinen beiden Enden durch dem Fachmann wohlbekannte Quetschdichtungen 2a, 2b verschlossen, wobei die elektrische Kontaktierung der Heizwendel 4 mittels elektrischer Leiter 5a, 5b und dünnen Molybdänfolien 6a, 6b erfolgt, die gasdicht in den Quetschdichtungen 2a, 2b eingebettet sind. Die Heizwendel 4 kann bei Temperaturen bis zu 1200°C bei hoher Lebensdauer betrieben werden. Für die Heizwendel 4 aus Carbonfaser-Material werden dabei Leistungen von über 90W/cm erreicht. Für eine Heizwendel aus einer Eisen-Chrom-Aluminium-Legierung in einem beidseitig offenen Hüllrohr werden Leistungen von über 50W/cm erreicht.

Figur 1 a zeigt den Querschnitt A - A' des Infrarotstrahlers 1 aus Figur 1. Es sind sechs Erhebungen 3 am Innendurchmesser des Hüllrohres 2 zu erkennen, die einen annähernd halbkreisförmigen Querschnitt aufweisen. Dadurch berührt die Heizwendel 4 die Erhebungen 3 lediglich an deren höchsten Stellen.

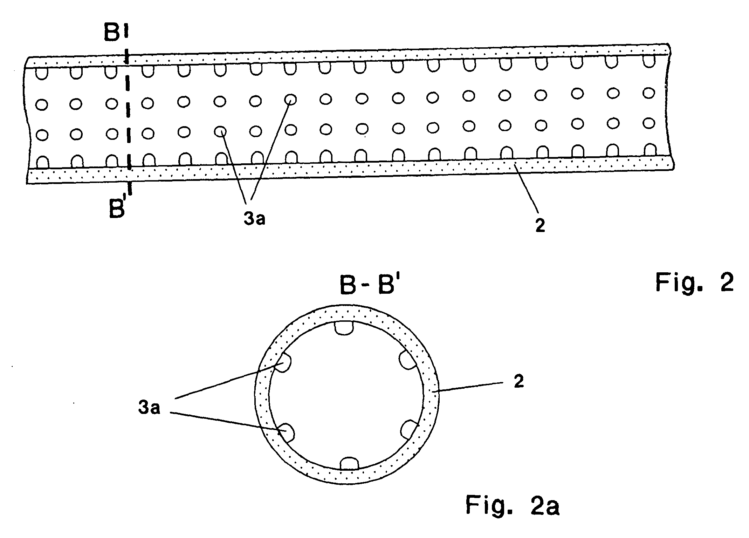

Figur 2 zeigt einen Längsschnitt durch ein für einen erfindungsgemäßen Infrarotstrahler geeignetes Hüllrohr 2. Das Hüllrohr 2 weist an seinem Innendurchmesser einzelne Erhebungen 3a aus Kieselglas auf, die sich in gerader Linie von einem Ende des Hüllrohres 2 zum anderen Ende des Hüllrohres 2 erstrecken.

Figur 2a zeigt den Querschnitt B - B' durch das Hüllrohr 2 aus Fig. 2. Es sind sechs Erhebungen 3a am Innendurchmesser des Hüllrohres 2 zu erkennen, die einen annähernd halbkreisförmigen Querschnitt aufweisen.

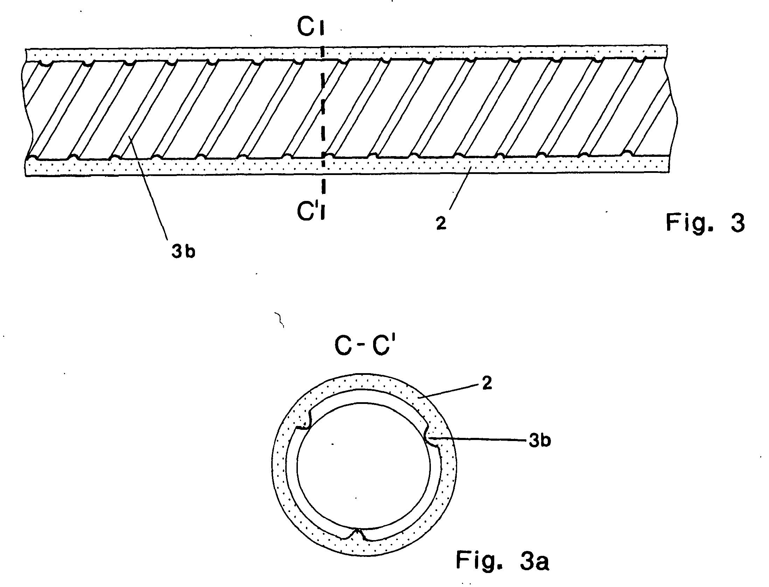

Figur 3 zeigt einen Längsschnitt durch ein weiteres für einen erfindungsgemäßen Infrarotstrahler geeignetes Hüllrohr 2. Das Hüllrohr 2 weist an seinem Innendurchmesser eine Erhebung 3b aus Kieselglas auf, die sich spiralförmig von einem Ende des Hüllrohres 2 zum anderen Ende des Hüllrohres 2 um die Längsachse des Hüllrohres 2 erstreckt.

Figur 3a zeigt den Querschnitt C - C' durch das Hüllrohr 2 aus Fig. 3.

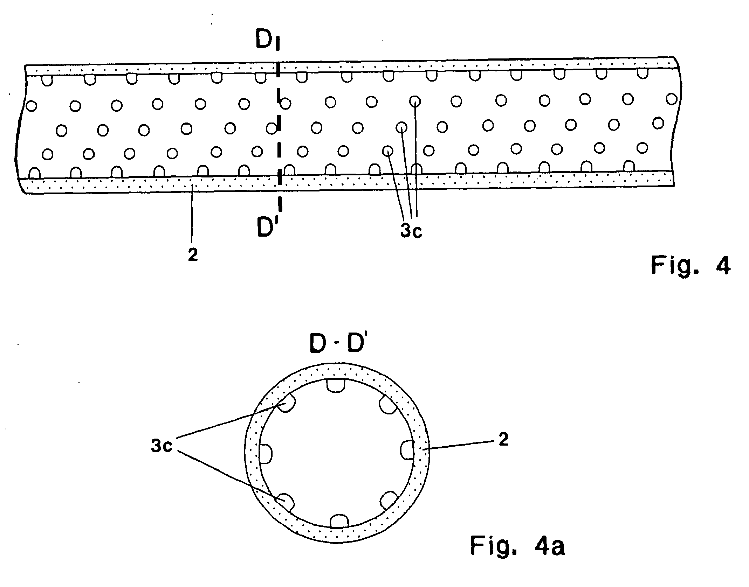

Figur 4 zeigt einen Längsschnitt durch ein weiteres für einen erfindungsgemäßen Infrarotstrahler geeignetes Hüllrohr 2. Das Hüllrohr 2 weist an seinem Innendurchmesser einzelne Erhebungen 3c aus Kieselglas auf, die sich spiralförmig von einem Ende des Hüllrohres 2 zum anderen Ende des Hüllrohres 2 um die Längsachse des Hüllrohres 2 erstrecken.

Figur 4a zeigt den Querschnitt D - D' durch das Hüllrohr 2 aus Fig. 4. Es sind acht Erhebungen 3c am Innendurchmesser des Hüllrohres 2 zu erkennen, die einen annähernd halbkreisförmigen Querschnitt aufweisen.

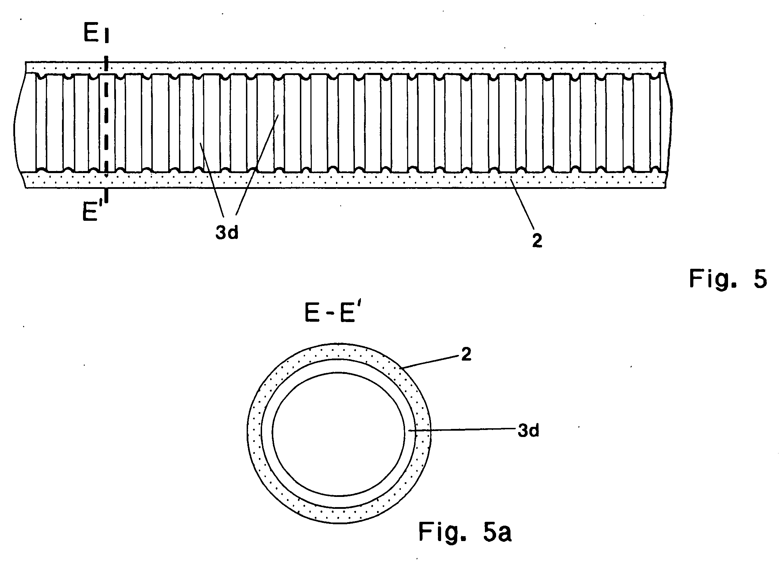

Figur 5 zeigt einen Längsschnitt durch ein weiteres für einen erfindungsgemäßen Infrarotstrahler geeignetes Hüllrohr 2. Das Hüllrohr 2 weist dabei an seinem Innendurchmesser Erhebungen 3d aus Kieselglas auf, die sich kreisförmig um die Längsachse des Hüllrohres 2 erstrecken.

Figur 5a zeigt den Querschnitt E - E' durch das Hüllrohr 2 aus Fig. 5.

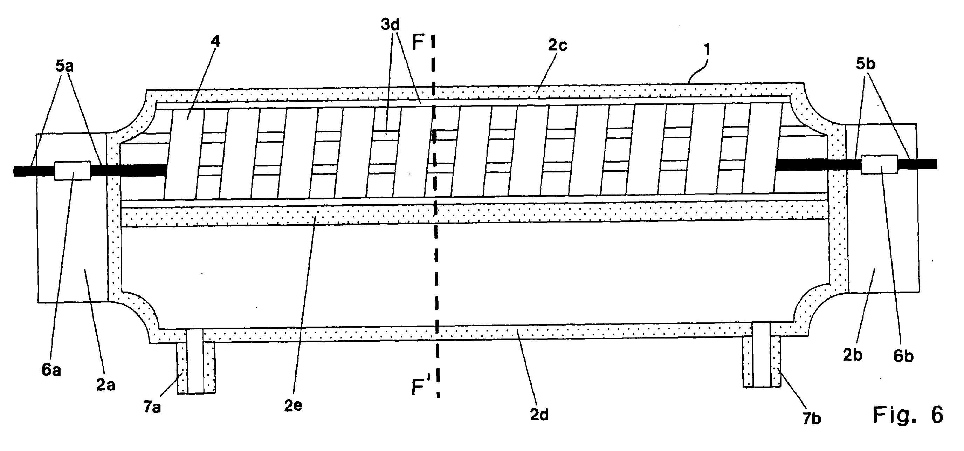

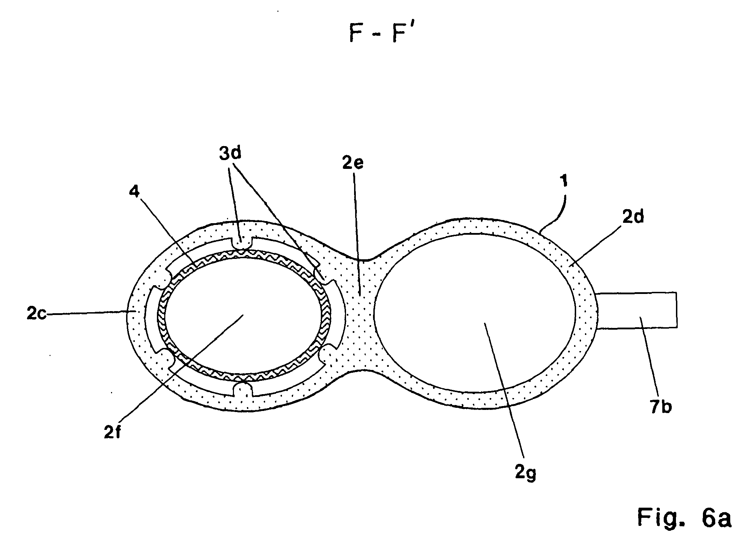

Figur 6 zeigt einen Längsschnitt durch einen erfindungsgemäßen Infrarotstrahler 1 mit einem als Zwillingsrohr 2c, 2d, 2e ausgestalteten Hüllrohr aus Kieselglas. Das Zwillingsrohr 2c, 2d, 2e weist dabei ein erstes Rohr 2c auf, das über den Steg 2e mit einem zweiten Rohr 2d verbunden ist. Das erste Rohr 2c weist an seinem Innendurchmesser Erhebungen 3d aus Kieselglas auf, die sich kreisförmig um die Längsachse des ersten Rohres 2c erstrecken. Eine Heizwendel 4 aus Carbonfaser-Material ist im ersten Rohr 2c derart angeordnet, dass der Außendurchmesser der Heizwendel 4 die Erhebungen 3d lediglich an deren höchsten Stellen berührt. Das zweite Rohr 2d weist Zu- und Abflussstutzen 7a, 7b auf, die dazu geeignet sind, einen Anschluss des zweiten Rohres 2d an eine Kühlwasserleitung vorzunehmen.

Die Rohre 2c, 2d sind an beiden Enden durch dem Fachmann wohlbekannte Quetschdichtungen 2a, 2b verschlossen, wobei die elektrische Kontaktierung der Heizwendel 4 mittels elektrischer Leiter 5a, 5b und dünnen Molybdänfolien 6a, 6b erfolgt, die gasdicht in den Quetschdichtungen 2a, 2b eingebettet sind.

Figur 6a zeigt den Querschnitt F - F' durch den Infrarotstrahler 1 aus Fig. 6. Es sind die Rohre 2c, 2d erkennbar, die im Bereich des Steges 2e miteinander verbunden sind. Dabei weist das Rohr 2c einen Rohrkanal 2f und das Rohr 2d einen Rohrkanal 2g auf. Im Rohr 2c ist eine Heizwendel 4 angeordnet, die die Erhebungen 3d lediglich an deren höchsten Stellen berührt.

Weitere Ausführungsformen des erfindungsgemäßen Infrarotstrahlers erschließen sich dem Durchschnittsfachmann in einfacher Weise.

## Patentansprüche

1. Infrarotstrahler mit mindestens einem elektrischen Heizleiter, welcher in einem zweiendigen, langgestreckten Hüllrohr aus Kieselglas angeordnet ist, wobei das Hüllrohr an seiner dem mindestens einen Heizleiter zugewandten Wandung mindestens eine Erhebung aus Kieselglas aufweist, die den Innendurchmesser des Hüllrohres lokal vermindert, wobei mindestens ein Heizleiter als eine Heizwendel (4) ausgebildet ist, das Hüllrohr (2) im Bereich der Heizwendel (4) einen konstanten Außendurchmesser aufweist, die Erhebung (3, 3a, 3b, 3c, 3d) auf ihrer der Heizwendel (4) zugewandten Oberfläche konvex geformt ist und die Heizwendel (4) die Erhebung (3, 3a, 3b, 3c, 3d) lediglich an der höchsten Stelle der Erhebung berührt.

2. Infrarotstrahler nach Anspruch 1, **dadurch gekennzeichnet, dass** die Erhebung(en) (3, 3a, 3b, 3c) mindestens eine Linie von einem Ende des Hüllrohres (2) zum anderen Ende des Hüllrohres (2) beschreibt / beschreiben.

3. Infrarotstrahler nach Anspruch 2, **dadurch gekennzeichnet, dass** die mindestens eine Linie aus einer einzigen, langgestreckten Erhebung (3, 3b) gebildet ist.

4. Infrarotstrahler nach Anspruch 3, **dadurch gekennzeichnet, dass** die linienförmige Erhebung (3, 3b) einen gleichbleibenden Querschnitt aufweist.

5. Infrarotstrahler nach Anspruch 3, **dadurch gekennzeichnet, dass** die linienförmige Erhebung (3, 3b) einen sich ändernden Querschnitt aufweist.

6. Infrarotstrahler nach Anspruch 2, **dadurch gekennzeichnet, dass** die mindestens eine Linie aus einer Anreihung von einzelnen Erhebungen (3a, 3c) gebildet ist.

7. Infrarotstrahler nach Anspruch 6, **dadurch gekennzeichnet, dass** die linienförmig angeordneten Erhebungen (3a, 3c) einen gleichbleibenden Querschnitt aufweisen.

8. Infrarotstrahler nach einem der Ansprüche 2 bis 7, **dadurch gekennzeichnet, dass** das Hüllrohr (2) eine Längsachse aufweist und dass die mindestens eine Linie parallel zur Längsachse des Hüllrohrs (2) verläuft.

9. Infrarotstrahler nach einem der Ansprüche 2 bis 7, **dadurch gekennzeichnet, dass** das Hüllrohr (2) eine Längsachse aufweist und dass die mindestens eine Linie wendelförmig um die Längsachse des Hüllrohrs (2) verläuft.

10. Infrarotstrahler nach Anspruch 1, **dadurch gekennzeichnet, dass** das Hüllrohr (2) eine Längsachse aufweist und dass die Erhebung(en) (3a, 3d) mindestens eine kreisförmige Linie um die Längsachse des Hüllrohrs (2) beschreibt / beschreiben.

11. Infrarotstrahler nach Anspruch 10, **dadurch gekennzeichnet, dass** die mindestens eine kreisförmige Linie aus einer einzigen, langgestreckten Erhebung (3d) gebildet ist.

12. Infrarotstrahler nach Anspruch 11, **dadurch gekennzeichnet, dass** die linienförmige Erhebung (3d) einen gleichbleibenden Querschnitt aufweist.

13. Infrarotstrahler nach Anspruch 11, **dadurch gekennzeichnet, dass** die linienförmige Erhebung (3d) einen sich ändernden Querschnitt aufweist.

14. Infrarotstrahler nach Anspruch 10, **dadurch gekennzeichnet, dass** die mindestens eine Linie aus einer Anreihung von einzelnen Erhebungen (3a) gebildet ist.

15. Infrarotstrahler nach Anspruch 14, **dadurch gekennzeichnet, dass** die linienförmig angeordneten Erhebungen (3a) einen gleichbleibenden Querschnitt aufweisen.

16. Infrarotstrahler nach einem der Ansprüche 1 bis 15, **dadurch gekennzeichnet, dass** die Heizwendel (4) aus einem.Wolframdraht gebildet ist.

17. Infrarotstrahler nach einem der Ansprüche 1 bis 15, **dadurch gekennzeichnet, dass** die Heizwendel (4) aus einem Kohlenstoff-Fasermaterial, aus Graphit oder Graphitpapier gebildet ist.

18. Infrarotstrahler nach einem der Ansprüche 1 bis 15, **dadurch gekennzeichnet, dass** die Heizwendel (4) aus einer Eisen-Chrom-Aluminium-Legierung, einer Nickel-Chrom-Legierung oder einer Nickel-Chrom-Eisen-Legierung gebildet ist.

19. Infrarotstrahler nach einem der Ansprüche 16 oder 17, **dadurch gekennzeichnet, dass** das Hüllrohr (2) an seinen Enden derart verschlossen ist, dass es den mindestens einen elektrischen Heizleiter gasdicht umschließt.

20. Infrarotstrahler nach Anspruch 18, **dadurch gekennzeichnet, dass** das Hüllrohr (2) an mindestens einem seiner Enden offen ist, so dass eine oxidierende Atmosphäre den mindestens einen elektrischen Heizleiter umgibt.

21. Infrarotstrahler nach einem der Ansprüche 1 bis 20, **dadurch gekennzeichnet, dass** das Hüllrohr (2) als ein Zwillingsrohr (2c, 2d, 2e) mit zwei Rohrkanälen (2f, 2g) ausgebildet ist, wobei das Hüllrohr (2) in mindestens einem der beiden Rohrkanäle (2f, 2g) die Heizwendel (4) aufweist.

## Claims

1. Infrared radiator with at least one electrical heating conductor which is provided in a dual-end, elongated jacket tube of quartz glass, the jacket tube comprising - on its wall facing the at least one heating conductor - at least one elevation of quartz glass which locally reduces the inside diameter of the jacket tube, with at least one heating conductor being designed as a heating coil (4), the jacket tube (2) having a constant outside diameter in the area of the heating coil (4), the elevation (3, 3a, 3b, 3c, 3d) being convex in form on its surface facing the heating coil (4), and the heating coil (4) touching the elevation (3, 3a, 3b, 3c, 3d) only at the highest point of the elevation.

2. Infrared radiator according to claim 1, **characterized in that** the elevation(s) (3, 3a, 3b, 3c) describe(s) at least one line from one end of the jacket tube (2) to the other end of the jacket tube (2).

3. Infrared radiator according to claim 2, **characterized in that** the at least one line is formed of one single, elongated elevation (3, 3b).

4. Infrared radiator according to claim 3, **characterized in that** the line elevation (3, 3b) has a constant cross-section.

5. Infrared radiator according to claim 3, **characterized in that** the line elevation (3, 3b) has a changing cross-section.

6. Infrared radiator according to claim 2, **characterized in that** the at least one line is formed of the stringing together of individual elevations (3a, 3c).

7. Infrared radiator according to claim 6, **characterized in that** the linearly arranged elevations (3a, 3c) have a constant cross-section.

8. Infrared radiator according to any one of the claims 2 to 7, **characterized in that** the jacket tube (2) has a longitudinal axis and that the at least one line is running parallel to the longitudinal axis of the jacket tube (2).

9. Infrared radiator according to any one of the claims 2 to 7, **characterized in that** the jacket tube (2) has a longitudinal axis and that the at least one line is running spirally around the longitudinal axis of the jacket tube (2).

10. Infrared radiator according to claim 1, **characterized in that** the jacket tube (2) has a longitudinal axis and that the elevation(s) (3a, 3d) describe(s) at least one circular line around the longitudinal axis of the jacket tube (2).

11. Infrared radiator according to claim 10, **characterized in that** the at least one circular line is formed of one single, elongated elevation (3d).

12. Infrared radiator according to claim 11, **characterized in that** the line elevation (3d) has a constant cross-section.

13. Infrared radiator according to claim 11, **characterized in that** the line elevation (3d) has a changing cross-section.

14. Infrared radiator according to claim 10, **characterized in that** the at least one line is formed of a stringing together of individual elevations (3a).

15. Infrared radiator according to claim 14, **characterized in that** the linearly arranged elevations (3a) comprise a constant cross-section.

16. Infrared radiator according to any one of the claims 1 to 15, **characterized in that** the heating coil (4) is formed of a tungsten filament.

17. Infrared radiator according to any one of the claims 1 to 15, **characterized in that** the heating coil (4) is formed of a carbon fiber material, of graphite, or of graphite paper.

18. Infrared radiator according to any one of the claims 1 to 15, **characterized in that** the heating coil (4) is formed of an iron/chromium/aluminum alloy, of a nickel/chromium alloy, or of a nickel/chromium/iron alloy.

19. Infrared radiator according to one of the claims 16 or 17, **characterized in that** the jacket tube (2) is closed at its ends such that it encloses, in a gas-tight manner, at least one electrical heating conductor.

20. Infrared radiator according to claim 18, **characterized in that** the jacket tube (2) is open in at least one of its ends so that an oxidizing atmosphere envelops the at least one electrical heating conductor.

21. Infrared radiator according to any one of the claims 1 to 20, **characterized in that** the jacket tube (2) is designed as a twin tube (2c, 2d, 2e) with two tube channels (2f, 2g), with the jacket tube (2) comprising the heating coil (4) in at least one of the two tube channels (2f, 2g).

## Revendications

1. Radiateur infrarouge avec au moins un conducteur chauffant électrique qui est placé dans un tube gainage très allongé à deux extrémités en verre de quartz, le tube de gainage comportant dans sa paroi dirigée vers le au moins un conducteur chauffant au moins un bossage en verre de quartz qui réduit localement le diamètre intérieur du tube de gainage, au moins un conducteur chauffant étant configuré comme un filament chauffant (4), le tube de gainage (2) présentant un diamètre extérieur constant dans la zone du filament chauffant (4), le bossage (3, 3a, 3b, 3c, 3d) ayant une forme convexe sur sa surface dirigée vers le filament chauffant (4) et le filament chauffant (4) venant en contact avec le bossage (3, 3a, 3b, 3c, 3d) uniquement au point le plus haut du bossage.

2. Radiateur infrarouge selon la revendication 1, **caractérisé en ce que** le (les) bossage(s) (3, 3a, 3b, 3c) décrit (décrivent) au moins une ligne entre une extrémité du tube de gainage (2) et l'autre extrémité du tube de gainage (2).

3. Radiateur infrarouge selon la revendication 2, **caractérisé en ce que** la au moins une ligne est formée par un seul bossage très allongé (3, 3b).

4. Radiateur infrarouge selon la revendication 3, **caractérisé en ce que** le bossage en forme de ligne (3, 3b) présente une section transversale constante.

5. Radiateur infrarouge selon la revendication 3, **caractérisé en ce que** le bossage en forme de ligne (3, 3b) présente une section transversale variable.

6. Radiateur infrarouge selon la revendication 2, **caractérisé en ce que** la au moins une ligne est formée par une rangée de bossages individuels (3a, 3c).

7. Radiateur infrarouge selon la revendication 6, **caractérisé en ce que** les bossages disposés en forme de ligne (3a, 3c) présentent une section transversale constante.

8. Radiateur infrarouge selon l'une quelconque des revendications 2 à 7, **caractérisé en ce que** le tube de gainage (2) présente un axe longitudinal et **en ce que** la au moins une ligne s'étend parallèlement à l'axe longitudinal du tube de gainage (2).

9. Radiateur infrarouge selon l'une quelconque des revendications 2 à 7, **caractérisé en ce que** le tube de gainage (2) présente un axe longitudinal et **en ce que** la au moins une ligne s'étend en forme d'hélice autour de l'axe longitudinal du tube de gainage (2).

10. Radiateur infrarouge selon la revendication 1, **caractérisé en ce que** le tube de gainage (2) présente un axe longitudinal et **en ce que** le(les) bossage(s) (3a, 3d) décrit (décrivent) au moins une ligne circulaire autour de l'axe longitudinal du tube de gainage (2).

11. Radiateur infrarouge selon la revendication 10, **caractérisé en ce que** la au moins une ligne circulaire est formée par un seul bossage très allongé (3d).

12. Radiateur infrarouge selon la revendication 11, **caractérisé en ce que** le bossage en forme de ligne (3d) présente une section transversale constante.

13. Radiateur infrarouge selon la revendication 11, **caractérisé en ce que** le bossage en forme de ligne (3d) présente une section transversale variable.

14. Radiateur infrarouge selon la revendication 10, **caractérisé en ce que** la au moins une ligne est formée par une rangée de bossages individuels (3a).

15. Radiateur infrarouge selon la revendication 14, **caractérisé en ce que** les bossages disposés en forme de ligne (3a) présentent une section transversale constante.

16. Radiateur infrarouge selon l'une quelconque des revendications 1 à 15, **caractérisé en ce que** le filament chauffant (4) est constitué d'un fil en tungstène.

17. Radiateur infrarouge selon l'une quelconque des revendications 1 à 15, **caractérisé en ce que** le filament chauffant (4) est constitué d'une matière en fibre de carbone, de graphite ou de papier graphité.

18. Radiateur infrarouge selon l'une quelconque des revendications 1 à 15, **caractérisé en ce que** le filament chauffant (4) est constitué d'un alliage de fer-chrome-aluminium, d'un alliage de nickel-chrome ou d'un alliage de nickel-chrome-fer.

19. Radiateur infrarouge selon l'une quelconque des revendications 16 ou 17, **caractérisé en ce que** le tube de gainage (2) est obturé à ses extrémités de telle manière qu'il entoure le au moins un conducteur chauffant électrique.

20. Radiateur infrarouge selon la revendication 18, **caractérisé en ce que** le tube de gainage (2) est ouvert à au moins l'une de ses extrémités de telle manière qu'une atmosphère oxydante entoure le au moins un conducteur chauffant électrique.

21. Radiateur infrarouge selon l'une quelconque des revendications 1 à 20, **caractérisé en ce que** le tube de gainage (2) est conçu comme un tube jumelé (2c, 2d, 2e) avec deux conduits de tube (2f, 2g), le tube de gainage (2) comportant le filament chauffant (4) dans au moins l'un des deux conduits de tube (2f, 2g).
